# EUROPÄISCHE PATENTSCHRIFT

(11) **EP 0 760 095 B1**
(45) Veröffentlichungstag und Bekanntmachung des Hinweises auf die Patenterteilung: **18.03.1998**
(21) Anmeldenummer: 95920036.1
(22) Anmeldetag: 13.05.1995
(51) Int. Cl.: G01N 27/30, C12M 1/34, B01L 3/00

(54) **OBJEKTTRÄGER FÜR MIKROSKOP**
SPECIMEN SLIDE FOR A MICROSCOPE
LAME PORTE-OBJET POUR MICROSCOPE

(30) Priorität: 17.05.1994 DE 4417079
(43) Veröffentlichungstag der Anmeldung: 05.03.1997
(73) Patentinhaber: Micronas Intermetall GmbH, 79108 Freiburg (DE); FRAUNHOFER-GESELLSCHAFT ZUR FÖRDERUNG DER ANGEWANDTEN FORSCHUNG E.V., 80636 München (DE)
(72) Erfinder: WOLF, Bernhard, D-79252 Stegen (DE); BAUMANN, Werner, D-77815 Bühl-Altschweier (DE); DUMBS, Alfred, D-79104 Freiburg (DE); SULZ, Gert, D-79224 Umkirch (DE); SIEBEN, Ulrich, D-79276 Reute (DE)
(86) Internationale Anmeldenummer: EP9501822
(87) Internationale Veröffentlichungsnummer: WO9531716

(56) Entgegenhaltungen:
- EP-A- 0 347 579
- EP-A- 0 545 284
- EP-A- 0 574 354
- DE-A- 4 236 421
- US-A- 4 974 952
- US-A- 5 278 048
- CLINICAL CHEMISTRY, Bd. 40, Nr. 9, September 1994 WINSTON US, Seiten 1805-1809, ARQUINT ET AL 'Micromachined analyzers on a silicon chip'

## Beschreibung

Die Erfindung bezieht sich auf einen Objektträger für Mikroskop, Kamera oder dergleichen Beobachtungseinrichtung, mit einem Aufnahmebereich für Testzellen, Gewebeschnitte oder dergleichen organischem Material, und mit einem gegenüber dem Aufnahmebereich kleineren Beobachtungsbereich für das organische Material, wobei der Objektträger zumindest im Beobachtungsbereich aus einem durchsichtigen Werkstoff besteht.

Solche Objektträger sind seit langem bekannt und ermöglichen es, eine in dem Aufnahmebereich des Objektträgers angeordnete organische oder biologische Substanz zusammen mit dem Objektträger auf einfache Weise auf dem Objekttisch eines Mikroskops zu positionieren. Dabei ist der Objektträger zumindest im Aufnahmebereich durchsichtig, damit die zu untersuchende Substanz beim Mikroskopieren mit einer Lichtquelle durchleuchtet werden kann.

Nachteilig ist dabei jedoch, daß zur Erzielung eines ausreichenden Vergrößerungsmaßstabes und zur Vermeidung von Abbildungsfehlern im Mikroskop, das zu untersuchende organische Material beim Mikroskopieren dicht benachbart zum Objektiv des Mikroskops angeordnet sein muß. Das organische Material, das beispielsweise eine Zellkultur sein kann, ist während des Mikroskopierens nur schwer zugänglich, so daß zusätzliche Untersuchungen an der unter dem Mikroskop nur relativ kurzlebigen Zellkultur kaum möglich sind.

Es besteht deshalb die Aufgabe, einen Objektträger der eingangs genannten Art zu schaffen, bei dem ein Objektiv, eine Beleuchtungseinrichtung oder dergleichen optisches Gerät dicht benachbart zum Aufnahmebereich angeordnet sein kann und bei dem dennoch während der cptischen Beobachtung eine weitergehende Untersuchung des organischen Materials möglich ist.

Die Lösung dieser Aufgabe besteht darin, daß der Objektträger innerhalb des Aufnahmebereiches, benachbart zu dem Beobachtungsbereich, wenigstens einen Dünnschicht- oder Planar-Sensor zur Messung physiologischer Parameter aufweist.

Dieser Sensor kann besonders kompakt aufgebaut und insbesondere in Planartechnik in den Objektträger integriert sein. Ähnlich wie eine integrierte Schaltung ist der Objektträger dadurch auch besonders einfach und kostengünstig als Großserienbauteil herstellbar.
In vorteilhafter Weise ermöglicht der zu dem Beobachtungsbereich benachbarte Dünnschicht- oder Planar-Sensor eine weitergehende Untersuchung des organischen Materials durch ein zu den optischen, beispielsweise durch Mikroskopieren erhaltenen Informationen paralleles Meßsignal. Wegen seiner geringen Bauhöhe kann der Sensor dabei in den Objektträger integriert oder auf der Oberfläche des Objektträgers eingeordnet sein, so daß Sensor und Objektträger eine Einheit bilden, die besonders gut handhabbar ist. Besonders vorteilhaft wirkt sich die geringe Bauhöhe des Sensors auch beim Mikroskopieren aus, da das Objektiv oder die Kondensorlinse eines Mikroskops bis dicht an das im Aufnahmebereich des Objektträgers angeordnete organische Material herangeführt werden kann, ohne daß das Objektiv oder die Kondensorlinse durch den Sensor behindert wird. Als Sensor kann beispielsweise ein Ionenkonzentrations-Sensor zur Messung von H⁺, Na⁺, Ca²⁺, K⁺ oder Cl -Ionen, ein Sauerstoffsensor, ein NO-Sensor, ein CO₂-Sensor, ein Temperatursensor, ein Interdigitalkondensator oder Referenz-Elektroden vorgesehen sein. Je nach Anwendung kann der Objektträger mit weiteren, insbesondere auch optischen Sensoren, wie z.B. Gitter-Kopplern, bestückt sein.

Mit dem erfindungsgemäßen Objektträger sind wichtige physikalisch-chemische Kenngrößen des zu untersuchenden organischen oder biologischen Materials einer Messung zugänglich, während gleichzeitig dessen Beobachtung, beispielsweise mit einem Mikroskop, möglich ist. Der Objektträger eignet sich besonders zur Überwachung und/oder Messung der Reaktion von biologischem Material auf verschiedene äußere chemische (z.B. Zytostatika, Ionenkonzentrationsveränderungen, Pharmaka, Umweltgifte, Waschsubstanzen), physikalische (z.B. Temperaturveränderungen, elektromagnetische Felder, Bestrahlungen, Beschleunigungen) sowie biologische (z.B. Antigene, Viren, Bakterien, Nährmediumsänderungen) Reize. Der Objektträger kann sowohl zu wissenschaftlichen Zwecken eingesetzt werden, als auch als Bio-Sensor Verwendung finden, beispielsweise bei der Gewässerkontrolle, wobei eine Probe des zu untersuchenden Wassers mit auf dem Objektträger befindlichen Testzellen in Berührung gebracht wird und die Reaktion der Testzellen mittels der Sensoren und gegebenenfalls durch optische Beobachtung ermittelt wird.

Eine Weiterbildung der Erfindung sieht vor, daß mehrere Sensoren am Umfang des Beobachtungsbereiches verteilt, vorzugsweise auf einem Kreis, dem Beobachtungsbereich benachbart angeordnet sind. In vorteilhafter Weise wird somit ein Objektträger zur Verfügung gestellt, der mehrere, sich sinnvoll ergänzende und gegebenenfalls gegenseitig kontrollierende Sensoren für Messungen von zellulären Prozessen aufweist. Dies ermöglicht insbesondere auch eine Korrelation der von den einzelnen Sensoren ermittelten Meßwerte, beispielsweise durch die Erstellung von Ortskurven, so daß die Zuverlässigkeit von Aussagen über das auf dem Objektträger befindliche organische Material erheblich verbessert ist. Die einzelnen Sensoren können mit einer Auswerteeinheit, beispielsweise einem Mikrocomputer, verbunden sein, die eine automatische Auswertung oder Analyse, insbesondere von komplexen physiologischen Vorgängen ermöglicht. Dabei können auch die mikroskopischen Daten berücksichtigt werden, wenn beispielsweise an dem Mikroskop eine CCD-Kamera angeschlossen ist, die mit einer Schnittstelle mit dem Mikrocomputer verbunden ist und der Mikrocomputer mit Methoden der Bildverarbeitung geeignete Kriterien zur Beurteilung der Mikroskopbilder ermittelt.

Zweckmäßigerweise sind an dem Objektträger elektrische Anschlüsse oder Kontaktflächen zum Abgreifen von Meßwerten, Meßsignalen und/oder zur Stromversorgung der Sensoren vorgesehen. Der Objektträger kann dann auf einfache Weise mit einer externen Auswerteeinheit zum Zwischenspeichern, Anzeigen oder Verarbeiten der mit den Sensoren detektierten Meßwerte verbunden werden. Dabei ist der Objektträger leicht austauschbar, so daß mehrere Objektträger nacheinander mit derselben Auswerteeinheit verbunden werden können.

Besonders günstig ist, wenn die Sensoren wenigstens teilweise in die Oberfläche des Objektträgers eingelassen sind und vorzugsweise bündig an diese anschließen. Im Aufnahmebereich des Objektträgers ergibt sich dadurch eine ebene Aufnahmefläche, auf der sich Testzellen oder dergleichen Zellkulturen gleichmäßig ausbreiten können. Inhomogenitäten in der Zellkultur, wie sie beispielsweise bei Unebenheiten oder an einer Stufe oder einem Absatz im Aufnahmebereich auftreten können, werden dadurch vermieden.

Vorteilhaft ist, wenn wenigstens ein Sensor als integrierte Schaltung mit Feldeffekttransistoren ausgebildet ist. Solche Sensoren weisen eine gute Sensitivität auf und sind mit Mitteln der Halbleitertechnik in Großserie preiswert herstellbar. Feldeffekttransistoren eignen sich besonders für eine Integration zur Zellpotentialmessung, wobei Interdigitalkondensatoren sowie weitere Sensoren (ISFET's) auf dem Chip integriert werden können.

Zweckmäßigerweise sind an der Oberfläche des Objektträgers Leiterbahnen vorgesehen, die beispielsweise aufgedruckt, aufgedampft und/oder wenigstens teilweise in die Oberfläche versenkt sind. Die Sensoren können dann auf einfache Weise, ähnlich wie bei einer Platine, mit den Leiterbahnen untereinander sowie mit den Kontaktflächen und evtl. weiteren, an dem Objektträger vorgesehenen elektronischen Bauelementen verbunden werden.

Eine Ausführungsform der Erfindung sieht vor, daß der Objektträger bis auf den Beobachtungsbereich im wesentlichen aus Silizium besteht, daß die Sensoren Silizium-Halbleiter sind und in das Silizium des Objektträgers integriert sind und daß der Objektträger im Beobachtungsbereich aus einem optisch durchsichtigen Werkstoff besteht. Dadurch ergibt sich ein besonders kostengünstig in Großserie herstellbarer Objektträger, bei dem mehrere, vorzugsweise alle Sensoren, in einem gemeinsamen Chip intergriert sein können. Der Chip kann dann im Beobachtungsbereich einen Durchbruch aufweisen, in den ein Glasfenster oder ein Kunststoffenster aus biologisch inertem hochtransparenten Material, beispielsweise Polycarbonat, einsetzbar ist.

Eine Weiterbildung der Erfindung sieht vor, daß der Objektträger zur Verarbeitung und/oder Auswertung der Sensorsignale einen Mikroprozessor, insbesondere einen Signalprozessor aufweist. Die einzelnen Anschlüsse für die Sensoren brauchen dann nicht getrennt nach außen geführt zu werden, sondern können auf dem Objektträger, beispielsweise mit Leiterbahnen, mit dem Mikro- oder Signalprozessor verbunden sein, der die Meßwerte der Sensoren digitalisiert und über eine beispielsweise serielle Schnittstelle, die an entsprechenden Anschlußkontakten an dem Objektträger zugänglich ist, nach außen weiterleitet. Der Objektträger braucht dann nur einige wenige externe Anschlüsse aufzuweisen, mit denen sämtliche auf dem Objektträger befindlichen Sensoren ansteuerbar und/oder auslesbar sind. Der Mikro- oder Signalprozessor ermöglicht dabei auch eine Meßwertverarbeitung, beispielsweise eine Filterung, Korrelation, Glättung oder Zwischenspeicherung von Meßwerten. Außerdem können in dem Prozessor Nichtliniaritäten der Sensoren, beispielsweise unter Berücksichtigung von Kennlinien oder einer möglicherweise vorhandenen Temperaturdrift, berücksichtigt werden.

Damit der photoelektrische Effekt bei Beleuchtung des Objektträgers in den Halbleitern der Sensoren keine Verfälschung der Meßwerte bewirkt, ist es vorteilhaft, wenn die Sensoren gegen Licht abgeschirmt sind und wenn hierzu insbesondere mit einer lichtundurchlässigen, vorzugsweise schwarzen Schicht vorgesehen ist. Wenn der Objektträger einen Kammerabschluß aufweist und ggf. eine solche Lichtabschirmung an der Ober- und an der Unterseite der Sensoren vorgesehen ist, eignet sich der Objektträger sowohl für Messungen an inversen, als auch an nichtinversen Mikroskopen. Das organische Material kann also wahlweise entweder an seiner dem Objektträger abgewandten Oberseite oder durch den Objektträger hindurch an seiner Unterseite mikroskopiert werden.

Damit das auf dem Objektträger befindliche organische Material möglichst genau auf Normaltemperatur (bei Säugerzellen 37,0 °C) gehalten werden kann, ist es vorteilhaft, wenn der Objektträger temperierbar ist.

Besonders günstig ist, wenn der Objektträger eine temperaturgeregelte Dünnschichtheizung aufweist und wenn diese vorzugsweise an einer dem Aufnahmebereich abgewandten Außenseite des Objektträgers vorgesehen ist. Bei einem als rechteckige Scheibe ausgebildeten Objektträger, bei dem der Aufnahmebereich an einer Flachseite angeordnet ist, ist also die Dünnschichtheizung an der dieser Flachseite abgewandten Flachseite vorgesehen. Der Objektträger und das darauf befindliche organische Material sind dadurch besonders gleichmäßig, praktisch an der gesamten Flachseite des Objektträgers, beheizbar. Zur Temperaturregelung der Dünnschichtheizung kann der Objektträger einen Temperatursensor aufweisen, der vorzugsweise innerhalb des Aufnahmebereiches und benachbart zu dem Beobachtungsbereich angeordnet ist.

Zum Fixieren des Objektträgers, beispielsweise am Objekttisch eines Mikroskops, kann eine Halterung vorgesehen sein. Vorteilhaft ist, wenn die Halterung temperierbar ist und in Gebrauchsstellung thermisch gut leitend, vorzugsweise mittels einer den Objektträger unterseitig berührenden wärmeübertragenden Haltefläche, mit dem Objektträger verbunden ist. Der Objektträger ist mittels der Halterung temperierbar und braucht deshalb keine eigene Heizung aufzuweisen.
Zur Temperierung des Objektträgers kann die Halterung einen Flüssigkeitskanal oder eine Hohlwand für Temperierflüssigkeit, mit einem Flüssigkeitszulauf und einem Flüssigkeitsablauf aufweisen. Der Flüssigkeitskanal läßt sich dann mit einer Umwälzpumpe mit einem thermostatisch geregelten Wasserbad oder einem Wasserkreislauf verbinden, so daß das zu untersuchende Zellmaterial sehr exakt auf einer vorgegebenen Temperatur gehalten werden kann.

Eine andere Ausführungsform sieht vor, daß die Halterung eine thermostatisch geregelte Elektroheizung aufweist. Eine solche Elektroheizung kann beispielsweise mit einem Halbleiter-Temperatursensor geregelt sein, mit dem die Temperatur des Halters und des damit verbundenen Objektträgers mit großer Genauigkeit eingestellt werden kann.

Vorteilhaft ist, wenn zum Abgreifen von elektrischen Signalen, insbesondere der Sensoren und/oder zur Stromeinspeisung ein auf die Kontaktflächen des Objektträgers aufsetzbares oder andrückbares Kontaktstück mit zu den Kontaktflächen des Objektträgers passenden Gegenkontakten vorgesehen ist. Mit einem solchen Kontaktstück können die externen elektrischen Verbindungen für mehrere Sensoren gleichzeitig, praktisch mit einem Handgriff, hergestellt werden.

Besonders vorteilhaft ist, wenn der Objektträger in ein genormtes DIL- oder PLCC IC-Gehäuse intergriert oder mit diesem verbunden ist. Das IC-Gehäuse des Objektträgers kann dann zum elektrischen Verbinden der Sensoren, beispielsweise mit einer externen Auswerteeinheit, auf einfache Weise in einen IC-Sockel eingesetzt werden. Dabei ist der Beobachtungsbereich des Objektträgers vorzugsweise mittig zur Flachseite des IC-Gehäuses angeordnet, damit der zwischen den Kontaktreihen des IC-Sockels vorhandene Freiraum für eine Beobachtung und/oder eine Beleuchtung des organischen Materials genutzt werden kann.

Eine Weiterbildung der Erfindung sieht vor, daß der Objektträger einen Kammerabschluß aufweist, der den Aufnahmebereich des Objektträgers seitlich dicht umschließt und zusammen mit diesem eine im wesentlichen geschlossene Kultivierungs-Kammer begrenzt, daß der Kammerabschluß ein Beobachtungs- und/oder Beleuchtungs-Fenster aufweist und wenigstens eine Einlaßöffnung sowie eine Auslaßöffnung für eine Nährflüssigkeit hat. In vorteilhafter Weise kann dadurch das in der Kammer befindliche organische Material, beispielsweise Testzellen, über einen längeren Zeitraum hinweg, beispielsweise länger als 10 Minuten, auf dem Mikroskop-Objekttisch unter definierten äußeren Bedingungen vital erhalten werden. Dies ermöglicht insbesondere eine noch bessere Untersuchung dynamisch sich verändernder Zellprozesse.

Zweckmäßigerweise ist vorgesehen, daß das Fenster einstückig mit dem Kammerabschluß ausgebildet ist und vorzugsweise aus biologisch inertem, hochtransparenten Kunststoff besteht. Der Kammerabschluß kann dann besonders einfach, beispielsweise als Polykarbonat-Körper mit etwa rechteckiger Außenkontur hergestellt sein, in den das Fenster integriert ist. Dabei wird bevorzugt ein thermisch gut leitender Kunststoff verwendet, damit das organische Material besser temperiert werden kann.

Besonders günstig ist, wenn das Fenster in einer in die Oberseite des Kammerabschlusses vorgesehenen Vertiefung angeordnet ist. In der Vertiefung kann dann eine Kondensorlinse mit hoher Apertur bis dicht an das organische Material herangeführt werden.

Vorteilhaft ist, wenn der Kammerabschluß Aufnahmevertiefungen hat, in die zusätzliche Sensoren mit Sensorhaltern lösbar einsetzbar, vorzugsweise einschraubbar oder einsteckbar sind. In die Kammer können dadurch zusätzliche Sensoren eingeführt werden, die auf dem Objektträger nicht vorgesehen sind. Diese Sensoren sind beliebig austauschbar, so daß die Kammer, je nach Anwendungsfall, mit der gewünschten Sensor-Konfiguration bestückt werden kann. Besonders vorteilhaft ist dabei, daß auch seltener benötigte Sensoren, bei denen sich eine Integration in den Objektträger nicht lohnt, zur Messung verwendet werden können.

Besonders günstig ist, wenn daß an einer der Kammer zugewandten Wandung des Kammerabschlusses Dünnschicht- oder Planar-Sensoren vorgesehen sind. Die Kammer kann dann als Miniatur-Kammer besonders kompakt aufgebaut werden, so daß auch an kleinste Mengen organischen Materials für eine Messung mit den Sensoren ausreichen. Dabei ermöglichen die an dem Objektträger angeordneten Sensoren, welche vorzugsweise direkt mit dem zu untersuchenden Zellmaterial in Berührung stehen oder von den Zellen bewachsen sind, eine Detektion der chemisch-physikalischen Eigenschaften des Zellmaterials, während während die in der Nährlösung angeordneten Dünnschicht- oder Planar-Sensoren des Kammerabschlusses besonders zur Messung von Nährflüssigkeitsparametern geeignet sind.

Zweckmäßigerweise ist vorgesehen, daß der Objektträger einen am Umfang des Aufnahmebereiches umlaufenden, nach oben überstehenden Halterand aufweist, der eine bis zum Aufnahmebereich reichende Öffnung umschließt, und daß der Kammerabschluß lösbar in die Öffnung einsetzbar ist. Die Kammer ist dann, beispielsweise zum Einbringen des organischen Materials nach Entfernen des Kammerabschlusses gut zugänglich. Am Umfang des Kammerabschlusses eine Dichtung vorgesehen sein, die in Funktionsstellung innenseitig an dem umlaufenden Rand anliegt und das Kammerinnere nach außen abdichtet.

Vorteilhaft ist, wenn der Objektträger im wesentlichen als rechteckige Scheibe ausgebildet ist und wenn der Aufnahmebereich an einer Flachseite des Objektträgers vorgesehen ist. Der Objektträger kann dann die Form eines handelsüblichen Standard-Objektträgers aufweisen, so daß dieser mit den an den Objekttischen der meisten Mikroskope vorgesehenen Haltevorrichtungen problemlos festlegbar ist.

Besonders günstig ist, wenn an dem Objektträger, vorzugsweise an dessen Oberseite, benachbart zu den schmalseitigen Rändern, wenigstens eine Aufnahmevertiefung zum Einsetzen eines Kontaktstückes vorgesehen ist. Die elektrischen Kontakte des Kontaktstückes sind dadurch in Funktionsstellung besonders genau zu den Gegenkontakten des Objektträgers ausgerichtet. Um ein versehentliches Herausziehen oder Lösen des Kontaktstückes aus der Aufnahmevertiefung zu verhindern, kann zwischen dem Kontaktstück und der Aufnahmevertiefung außerdem eine Rastverbindung vorgesehen sein.

Zweckmäßigerweise ist vorgesehen, daß der Aufnahmebereich zur Kultivierung nicht-adhärenter Zellen eine Oberflächenstruktur, insbesondere eine Lamellen- oder Gitterstruktur, aufweist. Nichtadhärente Zellen können dann im Durchflußbereich der Nährflüssigkeit nicht so leicht von dem Nährflüssigkeitsstrom mitgerissen oder weggespült werden.

Nachfolgend sind Ausführungsbeispiele der Erfindung anhand der Zeichnung näher erläutert.

Es zeigen in unterschiedlichen Maßstäben und zum Teil stärker schematisiert:

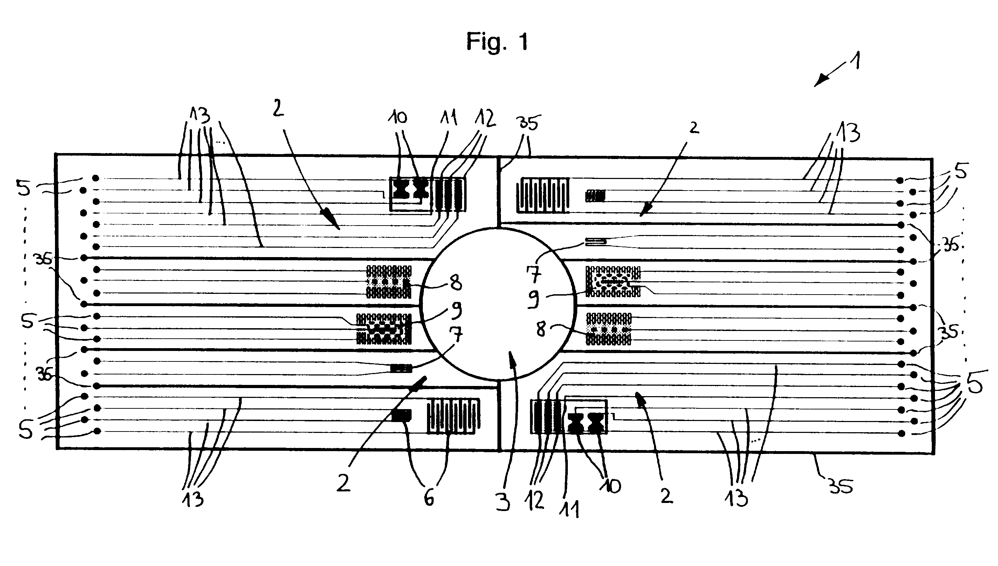

- Fig. 1: eine Aufsicht auf den erfindungsgemäßen Objektträger, welche die um den Beobachtungsbereich angeordneten Sensoren und die diese mit den Kontaktflächen verbindenden Leiterbahnen besonders gut erkennen läßt,
- Fig. 2: eine Aufsicht auf einen in eine beheizbare Halterung eingesetzten Objektträger, der einen am Umfang des Aufnahmebereiches umlaufenden Halterand für einen Kammerabschlusses aufweist und
- Fig. 3: einen Längsschnitt durch den in die Halterung eingesetzten Objektträger mit einem Kammerabschluß und mit beidseitig des Kammerabschlusses in Aufnahmevertiefungen eingesetzten Kontaktstücken für die elektrischen Anschlüsse der Sensoren.

Ein Objektträger 1 für ein Mikroskop, eine Kamera oder dergleichen Beobachtungseinrichtung weist einen Aufnahmebereich 2 für Testzellen oder dergleichen organisches Material sowie einen gegenüber dem Aufnahmebereich 2 kleineren Beobachtungsbereich 3 für das organische Material auf. Der Aufnahmebereich 2 und der Beobachtungsbereich 3 sind an der oberen Flachseite 4 des Objektträgers 1 angeordnet, wobei der Beobachtungsbereich 3 durch einen mittig zu der Flachseite 4 angeordnete, kreisrunden Teilbereich des Objektträgers 1 gebildet wird. Innerhalb des Beobachtungsbereiches 3 besteht der Objektträger 1 aus einem durchsichtigen, hochtransparenten Werkstoff, so daß das im Beobachtungsbereich 3 befindliche organische Material durch den Objektträger 1 hindurch beleuchtet und/oder mikroskopiert werden kann.

Der Objektträger 1 weist innerhalb des Aufnahmebereiches 2 und benachbart zu dem Beobachtungsbereich 3 folgende Dünnschichtsensoren auf: Vier Interdigitalkondensatoren 6, zwei Pt 100-Dünnschichttemperatursensoren 7, zwei NO-Sensoren, zwei O₂-Sensoren, zwei Zellpotentialsensoren mit FET's, zwei Temperatursensoren sowie sechs ISFET's. Selbstverständlich sind - je nach Anwendung - auch andere Sensor-Konfigurationen möglich.

In vorteilhafter Weise können durch die zahlreichen, an dem Objektträgers 1 vorgesehenen Sensoren 6 bis 12, die dicht benachbart zu dem organischen Material angeordnet sind oder mit diesem in Berührung stehen, während der Beobachtung mit einem Mikroskop Messungen der chemisch-physikalischen Eigenschaften des organischen Materials durchgeführt werden, die zusätzliche Informationen, beipsielsweise über zytophysiologische Abläufe in dem organischen Material ermöglichen. Dabei kann eine Kondensorlinse eines Mikrokops oder ein Objektiv mit hoher Apertur dicht benachbart zu dem Beobachtungsbereich 3 angeordnet sein, ohne daß dieses durch die Sensoren 6 bis 12 behindert wird. Durch Korrelation der mit den einzelnen Sensoren gewonnenen Meßergebnisse sowie gegebenenfalls durch Vergleich mit den durch optische Beobachtung erhaltenen Informationen kann die Zuverlässigkeit von Aussagen über den Zustand des auf dem Objektträger 1 befindlichen immobilisierten biologischen oder organischen Zellmaterials verbessert werden. Der Objektträger 1 kann sowohl zu Forschungszwecken, als auch als Bio-Sensor verwendet werden.

Die Sensoren 6 bis 12 sind zumindest teilweise in die Oberfläche des Objektträgers 1 eingelassen und schließen vorzugsweise bündig an diese an. Auf dem Objektträger 1 befindliche Zellkulturen können sich dadurch gleichmäßig über den gesamten Aufnahmebereich 2 ausbilden, ohne daß sie durch Unebenheiten, Absätze oder Stufen im Aufnahmebereich 2 behindert werden.

Die Sensoren 6 bis 12 sind in Dünnschicht-Technik als Integrierte Schaltungen mit Feldeffekttransistoren ausgebildet, die mit Methoden der Halbleitertechnik in Großserie kostengünstig hergestellbar sind.

An dem Objektträger 1 sind ferner elektrische Kontaktflächen 5 zum Abgreifen von Meßwerten, Meßsignalen und/oder zur Stromversorgung der Sensoren 6 bis 12 vorgesehen. Die Kontaktflächen 5 sind an den schmalseitigen Rändern der oberen Flachseite 4 des Objektträgers 1 angeordnet und sind mit Leiterbahnen 13 elektrisch mit den Sensoren 6 bis 12 verbunden. Die Leiterbahnen 13 können beispielsweise aufgedruckt, aufgedampft oder mit photo-chemischen Verfahren auf die Oberfläche des Objektträgers 1 aufgebracht sein. Zwischen den Sensoren 6 bis 12 sowie um den Beoachtungsbereich 3 herum sind Masse-Verbindungen 35 vorgesehen, die eine Abschirmung der Sensoren 6 bis 12 bewirken.

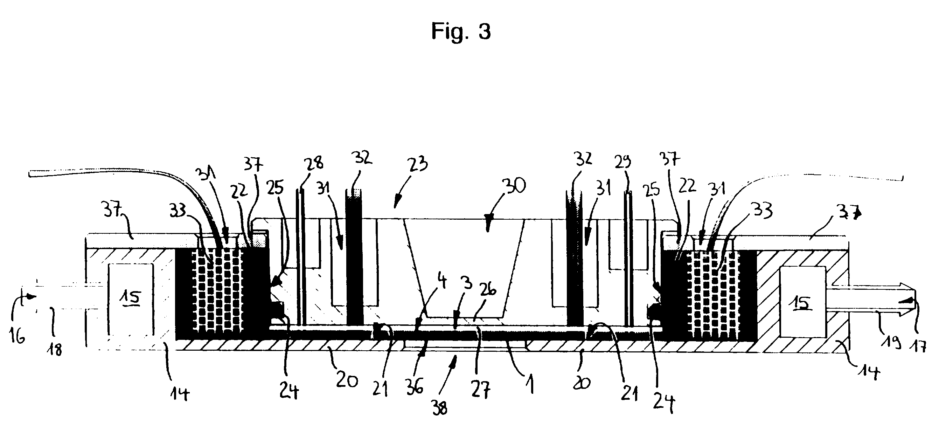

Zum Fixieren des Objektträgers 1 ist eine Halterung 14 vorgesehen, die eine Aufnahmevertiefung aufweist, in die der Objektträger 1 einsetzbar ist (Fig. 3). Die Halterung 14 hat einen am Außenumfang umlaufenden Flüssigkeitskanal 15 mit einem Flüssigkeitszulauf 16 und einem Flüssigkeitsablauf 17. Der Flüssigkeitszulauf 16 und Flüssigkeitsablauf 17 weisen jeweils einen Anschlußstutzen 18, 19 auf, mit denen der Flüssigkeitskanal 15 mit einem thermostatisch geregelten Wasserkreislauf oder einem temperierbaren Wasserbad verbindbar ist. Die Halterung 14 und der damit verbundene Objektträger 1 können dadurch sehr exakt temperiert werden, so daß das organische Material auf einer praktisch konstanten Temperatur von beipsielsweise 37,0 °C gehalten werden kann. Die Halterung 14 besteht im wesentlichen aus einem thermisch gut leitenden, korrosionbeständigen Werkstoff (Edelstahl) und weist im Bereich ihrer Aufnahmevertiefung einen Haltesteg 20 mit einer wärmeübertragenden Haltefläche 21 auf, die in Funktionsstellung großflächig an der unteren Flachseite 36 des Objektträgers 1 anliegt und daher eine gute Wärmeübertragung auf den Objektträger 1 ermöglicht. Unterhalb des Beobachtungsbereichs 3 ist in dem Haltesteg 20 ein kreisrunder Durchbruch 38 vorgesehen.

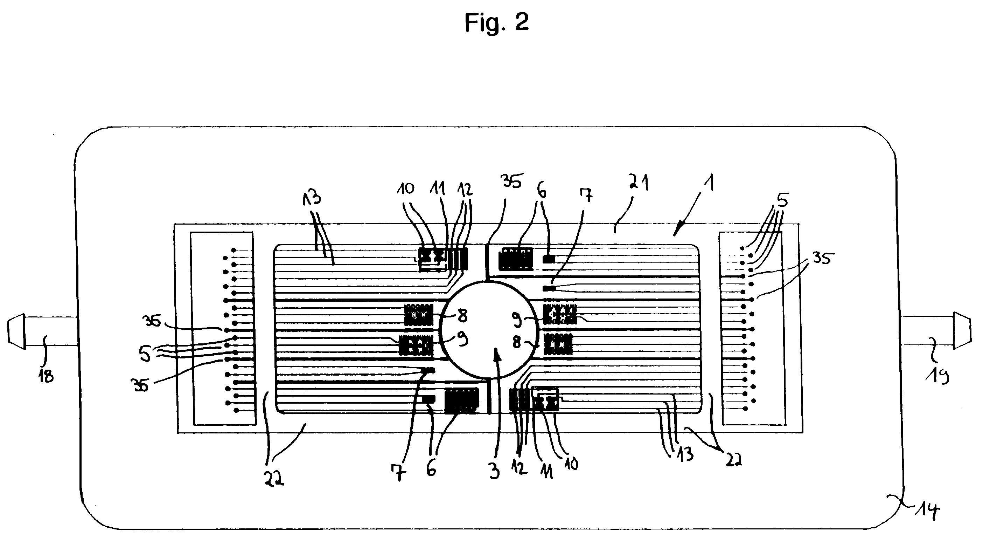

Bei dem in Fig. 2 und 3 dargestellten Ausführungsbeispiel weist der Objektträger 1 einen am Umfang des Aufnahmebereiches 2 umlaufenden, nach oben über den Aufnahmebereich 2 überstehenden Halterand 22 auf, der eine bis zum Aufnahmebereich 2 reichende Öffnung umschließt, in die ein Kammerabschluß 23 einsetzbar ist. Der Halterand 22 schließt seitlich zum Aufnahmebereich 2 dicht an den Objektträger 1 an und kann beispielsweise durch einen aufgeklebten Kunststoffrahmen oder als aufgespritztes Kunststoffteil realisiert sein. Der Kammerabschluß 23 hat ferner einen in einer Nut umfangsseitig umlaufenden Dichtring 24, der in Funktionsstellung gegen die Innenfläche 25 des Halterandes 22 abdichtet, so daß zwischen dem Kammerabschluß 23 und dem Objektträger 1 ein im wesentlichen geschlossenes Kammervolumen eingeschlossen wird.

Der Kammerabschluß 23 weist ferner ein benachbart zu dem Beobachtungsbereich 3 angeordnetes Beobachtungs- und/oder Beleuchtungs-Fenster 26 auf, durch das das in der Kammer 27 befindliche organische Material, beispielsweise mit einem Mikroskop, betrachtet werden kann. Der Kammerabschluß 23 hat außerdem eine Einlaßöffnung 28 und eine Auslaßöffnung 29 zum Austauschen einer in der Kammer 27 vorgesehenen Nährflüssigkeit. Das in der Kammer 27 befindliche Zell- oder Gewebematerial kann dadurch über eine längere Zeit, unter praktisch konstanten äußeren Bedingungen, vital erhalten werden. Zur Reduzierung der von der Nährflüssigkeitsströmung auf die in der Kammer 2 befindlichen Zellen ausgeübten Scherkräfte können weitere Einlaßöffnungen 28 und/oder Auslaßöffnungen 29 vorgesehen sein.

Das Fenster 26 ist einstückig mit dem Kammerabschluß 23 ausgebildet und besteht aus biologisch inertem hochtransparentem Kunststoff. Der Kammerabschluß 23 ist dadurch besonders kostengünstig herstellbar. Das Fenster 26 ist in einer in die Oberseite des Kammerabschlusses 23 eingelassenen Vertiefung 30 angeordnet, so daß eine Kondensorlinse oder ein Objektiv mit großem Aperturwinkel bis dicht an das zu untersuchende Zellmaterial herangeführt werden kann.

Der Kammerabschluß 23 hat außerdem Aufnahmeöffnungen 31, in die zusätzliche Sensoren 32 mit Sensorhaltern lösbar einsetzbar sind. Die aus dem Objektträger 1, dem Halterand 22 und dem Kammerabschluß 23 gebildete Vorrichtung ist dadurch noch fllexibler einsetzbar, wobei die Sensorkonfiguration auf einfache Weise durch Austauschen der in die Aufnahmeöffnungen 31 eingesetzten Sensoren 32 an die jeweilige Meßaufgabe angepasst werden kann. Dabei können zum Verschließen nichtbenötigter Aufnahmeöffnungen 31 Verschlußstopfen vorgesehen sein.

Zum Abgreifen von elektrischen Signalen, insbesondere der Sensoren 6 bis 12 und/oder zur Stromeinspeisung weist der Objektträger 1 zwei Kontaktstücke 33 mit zu den Kontaktflächen 5 des Objektträgers 1 passenden Gegenkontakten auf.

Der Objektträger 1 hat ferner an seiner Oberseite, benachbart zu seinen schmalseitigen Enden, jeweils eine Aufnahmevertiefung 34 für ein Kontaktstück 33. Die Kontaktstücke 33 können dadurch auf einfache Weise relativ zu dem Objektträger 1 positioniert werden, wobei die Kontaktflächen 5 des Objektträgers 1 mit den Gegenkontakten des Kontaktstückes 33 in Berührung geraten. Die Kontaktstücke 33 weisen einen asymmetrischen Querschnitt auf, der ein versehentliches spiegelverkehrtes Einsetzen der Kontaktstücke 33 in die Aufnahmeöffnungen 31 verhindert.

An der Oberseite der Halterung 14 ist eine Abdeckung 37 vorgesehen, die bereichsweise über den Halterand 22 und die in die Aufnahmevertiefungen 34 eingesetzten Kontaktstücke 33 übersteht. Die Abdeckung 37 ist mit einer Halteklammer (nicht dargestellt) mit der Halterung 14 verbindbar, so daß der Objektträger 1, die Kontaktstücke 33, die Halterung 14 und die Abdeckung 37 dann formschlüssig zu einer leicht transportierbaren Einheit verbunden sind.

## Patentansprüche

1. Objektträger für Mikroskop, Kamera oder dergleichen Beobachtungseinrichtung, mit einem Aufnahmebereich (2) für Testzellen, Gewebeschnitte oder dergleichen organischem Material, und mit einem gegenüber dem Aufnahmebereich (2) kleineren Beobachtungsbereich (3) für das organische Material, wobei der Objektträger (1) zumindest im Beobachtungsbereich (3) aus einem durchsichtigen Werkstoff besteht, **dadurch gekennzeichent**, daß der Objektträger (1) innerhalb des Aufnahmebereiches (2) benachbart zu dem Beobachtungsbereich (3) wenigstens einen Dünnschicht- oder Planar-Sensor (6 bis 12) zur Messung physiologischer Parameter aufweist.

2. Objektträger nach Anspruch 1, dadurch gekennzeichnet, daß mehrere Sensoren (6 bis 12) am Umfang des Beobachtungsbereiches (3) verteilt, vorzugsweise auf einem Kreis, dem Beobachtungsbereich (3) benachbart angeordnet sind.

3. Objektträger nach Anspruch 1 oder 2, dadurch gekennzeichnet, daß an dem Objektträger (1) elektrische Anschlüsse oder Kontaktflächen (5) zum Abgreifen von Meßwerten, Meßsignalen und/oder zur Stromversorgung der Sensoren (6 bis 12) vorgesehen sind.

4. Objektträger nach einem der Ansprüche 1 bis 3, dadurch gekennzeichnet, daß die Sensoren (6 bis 12) wenigstens teilweise in die Oberfläche des Objektträgers (1) eingelassen sind und vorzugsweise bündig an diese anschließen.

5. Objektträger nach einem der Ansprüche 1 bis 4, dadurch gekennzeichnet, daß wenigstens ein Sensor (6 bis 12) als integrierte Schaltung mit Feldeffekttransistoren ausgebildet ist.

6. Objektträger nach einem der Ansprüche 1 bis 5, dadurch gekennzeichnet, daß an der Oberfläche des Objektträgers (1) Leiterbahnen (13) vorgesehen sind, die beispielsweise aufgedruckt, aufgedampft und/oder wenigstens teilweise in die Oberfläche versenkt sind.

7. Objektträger nach einem der Ansprüche 1 bis 6, dadurch gekennzeichnet, daß der Objektträger (1) bis auf den Beobachtungsbereich (3) im wesentlichen aus Silizum besteht, daß die Sensoren (6 bis 12) Silizium-Halbleiter sind und in das Silizium des Objektträgers (1) integriert sind und daß der Objektträger (1) im Beobachtungsbereich (3) aus einem optisch durchsichtigen Werkstoff besteht.

8. Objektträger nach einem der Ansprüche 1 bis 7, dadurch gekennzeichnet, daß die Sensoren (6 bis 12) gegen Licht abgeschirmt sind und daß dazu insbesondere eine lichtundurchlässige, vorzugsweise schwarzen Schicht vorgesehen ist.

9. Objektträger nach einem der Ansprüche 1 bis 8, dadurch gekennzeichnet, daß der Objektträger (1) zur Verabeitung und/oder Auswertung der Sensorsignale einen Mikroprozessor, insbesondere einen Signalprozessor aufweist.

10. Objektträger nach einem der Ansprüche 1 bis 9, dadurch gekennzeichnet, daß der Objektträger (1) temperierbar ist.

11. Objektträger nach einem der Ansprüche 1 bis 10, dadurch gekennzeichnet, daß der Objektträger (1) eine temperaturgeregelte Dünnschichtheizung aufweist und daß diese vorzugsweise an einer dem Aufnahmebereich (2) abgewandten Außenseite des Objektträgers (1) vorgesehen ist.

12. Objektträger nach einem der Ansprüche 1 bis 11, dadurch gekennzeichnet, daß zum Fixieren des Objektträgers (1) eine Halterung (14) vorgesehen ist.

13. Objektträger nach einem der Ansprüche 1 bis 12, dadurch gekennzeichnet, daß die Halterung (14) temperierbar ist und in Gebrauchsstellung thermisch gut leitend, vorzugsweise mittels einer den Objektträger (1) unterseitig berührenden wärmeübertragenden Haltefläche (21), mit dem Objektträger (1) verbunden ist.

14. Objektträger nach einem der Ansprüche 1 bis 13, dadurch gekennzeichnet, daß die Halterung (14) einen Flüssigkeitskanal (15) oder eine Hohlwand für Temperierflüssigkeit, mit einem Flüssigkeitszulauf (16) und einem Flüssigkeitsablauf (17) aufweist.

15. Objektträger nach einem der Ansprüche 1 bis 14, dadurch gekennzeichnet, daß die Halterung (14) eine thermostatisch geregelte Elektroheizung aufweist.

16. Objektträger nach einem der Ansprüche 1 bis 15, dadurch gekennzeichnet, daß zum Abgreifen von elektrischen Signalen, insbesondere der Sensoren (6 bis 12), und/oder zur Stromeinspeisung ein auf die Kontaktflächen (5) des Objektträgers (1) aufsetzbares oder andrückbares Kontaktstück (33) mit zu den Kontaktfächen (5) des Objektträgers (1) passenden Gegenkontakten vorgesehen ist.

17. Objektträger nach einem der Ansprüche 1 bis 16, dadurch gekennzeichnet, daß der Objektträger (1) in ein genormtes DIL- oder PLCC IC-Gehäuse integriert oder mit diesem verbunden ist.

18. Objektträger nach einem der Ansprüche 1 bis 17, dadurch gekennzeichnet, daß ein Kammerabschluß (23) vorgesehen ist, der den Aufnahmebereich (2) des Objektträgers (1) seitlich dicht umschließt und zusammen mit diesem eine im wesentlichen geschlossene Kultivierungs-Kammer (27) begrenzt, daß der Kammerabschluß (23) ein Beobachtungs- und/oder Beleuchtungs-Fenster (26) aufweist und wenigstens eine Einlaßöffnung (28) sowie wenigstens eine Auslaßöffnung (29) für eine Nährflüssigkeit hat.

19. Objektträger nach einem der Ansprüche 1 bis 18, dadurch gekennzeichnet, daß das Fenster (26) einstückig mit dem Kammerabschluß (23) ausgebildet ist und vorzugsweise aus biologisch inertem, hochtransparenten Kuststoff besteht.

20. Objektträger nach einem der Ansprüche 1 bis 19, dadurch gekennzeichnet, daß das Fenster in einer in der Oberseite des Kammerabschlusses (23) vorgesehenen Vertiefung angeordnet ist.

21. Objektträger nach einem der Ansprüche 1 bis 20, dadurch gekennzeichnet, daß der Kammerabschluß (23) Aufnahmeöffnungen (31) hat, in die zusätzliche Sensoren (32) mit Sensorhaltern lösbar einsetzbar, vorzugsweise einschraubbar oder einsteckbar sind.

22. Objektträger nach einem der Ansprüche 1 bis 21, dadurch gekennzeichnet, daß an einer der Kammer (27) zugewandten Wandung des Kammerabschlusses (23) Dünnschicht- oder Planar-Sensoren vorgesehen sind.

23. Objektträger nach einem der Ansprüche 1 bis 22, dadurch gekennzeichnet, daß zum Verschließen nicht benötigter Aufnahmeöffnungen (31) Verschlußstopfen vorgesehen sind.

24. Objektträger nach einem der Ansprüche 1 bis 23, dadurch gekennzeichnet, daß der Objektträger (1) einen am Umfang des Aufnahmebereiches (2) umlaufenden, nach oben überstehenden Halterand (22) aufweist, der eine bis zum Aufnahmebereich (2) reichende Öffnung umschließt, und daß der Kammerabschluß (23) lösbar in die Öffnung einsetzbar ist.

25. Objektträger nach einem der Ansprüche 1 bis 24, dadurch gekennzeichnet, daß der Objektträger (1) im wesentlichen als rechteckige Scheibe ausgebildet ist und daß der Aufnahmebereich (2) an einer Flachseite (4) der Scheibe vorgesehen ist.

26. Objektträger nach einem der Ansprüche 1 bis 25, dadurch gekennzeichnet, daß an dem Objektträger (1), vorzugsweise an dessen Oberseite, benachbart zu den schmalseitigen Rändern, wenigstens eine Aufnahmevertiefung (34) zum Einsetzen eines Kontaktstückes vorgesehen ist.

27. Objektträger nach einem der Ansprüche 1 bis 26, dadurch gekennzeichnet, daß der Aufnahmebereich (2) zur Kultivierung nicht-adhärenter Zellen eine Oberflächenstruktur, insbesondere eine Lamellen- oder Gitterstruktur, aufweist.

## Claims

1. A specimen slide for a microscope, camera or other such observation device, having a receiving area (2) for cell or tissue samples or other such organic material and further having a smaller observation area (3) for the organic material, whereby the specimen slide (1) at least in the observation area (3) is made of transparent material, **characterized in that** within the receiving area (2), adjacent to the observation area (3), the specimen slide (1) has at least one thin-film sensor or planar sensor (6 to 12) for measuring physiological parameters.

2. A specimen slide as claimed in claim 1, characterized in that a plurality of sensors (6 to 12) are distributed over the periphery of the observation area (3), preferably on a circle, adjacent to the observation area (3).

3. A specimen slide as claimed in claim 1 or claim 2, characterized in that the specimen slide (1) is provided with electric connections or contact surfaces (5) for picking off measured values, measuring signals and/or for the power supply of the sensors (6 to 12).

4. A specimen slide as claimed in any one of claims 1 to 3, characterized in that the sensors (6 to 12) are at least partly sunk in, and are preferably flush with, the surface of the specimen slide (1).

5. A specimen slide as claimed in any one of claims 1 to 4, characterized in that at least one sensor (6 to 12) takes the form of an integrated circuit with field-effect transistors.

6. A specimen slide as claimed in any one of claims 1 to 5, characterized in that the surface of the specimen slide (1) is provided with strip conductors (13) which are for instance printed, vapour-deposited and/or at least partly sunk in the surface.

7. A specimen slide as claimed in any one of claims 1 to 6, characterized in that the specimen slide (1), except for the observation area (3), essentially consists of silicon, that the sensors (6 to 12) are silicon semiconductors and are integrated in the silicon of the specimen slide (1) and that in the observation area (3) the specimen slide (1) consists of an optically transparent material.

8. A specimen slide as claimed in any one of claims 1 to 7, characterized in that the sensors (6 to 12) are shielded against light and that for this purpose in particular a layer impervious to light, preferably a black layer, is provided.

9. A specimen slide as claimed in any one of claims 1 to 8, characterized in that the specimen slide (1) has a microprocessor, particularly a signal processor, for processing and/or evaluating the sensor signals.

10. A specimen slide as claimed in any one of claims 1 to 9, characterized in that the specimen slide (1) is temperature-regulable.

11. A specimen slide as claimed in any one of claims 1 to 10, characterized in that the specimen slide (1) has a temperature-controlled thin-film heating and that the same is preferably provided on the specimen slide (1) at an outer surface thereof facing away from the receiving area (2).

12. A specimen slide as claimed in any one of claims 1 to 11, characterized in that a holder (14) is provided for fixing the specimen slide (1).

13. A specimen slide as claimed in any one of claims 1 to 12, characterized in that the holder (14) is temperature-regulable and in the position of use is connected with good thermal conductivity to the specimen slide (1), preferably by means of a heat-transferring surface (21) contacting the underside of the specimen slide (1).

14. A specimen slide as claimed in any one of claims 1 to 13, characterized in that the holder (14) has a liquid duct (15) or a hollow wall for temperature-regulating liquid, with a liquid inlet (16) and a liquid outlet (17).

15. A specimen slide as claimed in any one of claims 1 to 14, characterized in that the holder (14) has a thermostatically controlled electric heating.

16. A specimen slide as claimed in any one of claims 1 to 15, characterized in that for picking off electric signals, particularly of the sensors 6 to 12), and/or for feeding current, a contact member (33) which is adapted to be attached or pressed onto the contact surfaces (5) of the specimen slide (1) and has contacts mating the contact surfaces (5) of the specimen slide (1) is provided.

17. A specimen slide as claimed in any one of claims 1 to 16, characterized in that the specimen slide (1) is integrated in or connected to a standardized DIL package or PLCC IC package.

18. A specimen slide as claimed in any one of claims 1 to 17, characterized in that a chamber closure (23) is provided laterally tightly surrounding the receiving area (2) of the specimen slide (1) and therewith defining a substantially closed cultivation chamber (27), that the chamber closure (23) has a viewing and/or illuminating window (26) and at least one inlet opening (28) and at least one outlet opening (29) for a nutrient liquid.

19. A specimen slide as claimed in any one of claims 1 to 18, characterized in that the window (26) is integral with the chamber closure (23) and preferably consists of biologically inert, highly transparent plastic.

20. A specimen slide as claimed in any one of claims 1 to 19, characterized in that the window is arranged in a depression provided in the upper surface of the chamber closure (23).

21. A specimen slide as claimed in any one of claims 1 to 20, characterized in that the chamber closure (23) has locating openings (31) into which additional sensors (32) with sensor holders can be detachably placed, preferably screwed or plugged in.

22. A specimen slide as claimed in any one of claims 1 to 21, characterized in that thin-film sensors or planar sensors are provided on a chamber closure (23) wall facing the chamber (27).

23. A specimen slide as claimed in any one of claims 1 to 22, characterized in that stoppers are provided for stoppering locating openings (31) not required.

24. A specimen slide as claimed in any one of claims 1 to 23, characterized in that the specimen slide (1) has running round the periphery of the receiving area (2) a holding edge (22) which projects upwardly and surrounds an opening extending to the receiving area (2), and that the chamber closure (23) is adapted to be detachably inserted in the opening.

25. A specimen slide as claimed in any one of claims 1 to 24, characterized in that the specimen slide (1) essentially takes the form of a rectangular wafer and that the receiving area (2) is provided on one face (4) of the wafer.

26. A specimen slide as claimed in any one of claims 1 to 25, characterized in that at least one locating recess (34) for inserting a contact member is provided on the specimen slide (1), preferably on the upper side thereof, adjacent to the narrow edges.

27. A specimen slide as claimed in any one of claims 1 to 26, characterized in that for cultivation of non-adherent cells the receiving area (2) has a surface structure, particularly a lamellar or lattice structure.

## Revendications

1. Lame porte-objet pour microscope, caméra ou dispositifs d'observation analogues, comportant une zone de prise de vue (2) pour des cellules de test, des morceaux de tissu ou une matière organique analogue et une zone d'observation (3) plus petite par rapport à la zone de prise de vue (2) et destinée à la matière organique, la lame porte-objet (1) étant constituée, au moins dans la zone d'observation (3), d'une matière transparente, caractérisée en ce que la lame porte-objet (1) comporte, à l'intérieur de la zone de prise de vue (2), au voisinage de la zone d'observation (3), au moins un capteur à couche mine ou à structure plane (6 à 12) destiné à la mesure de paramètres physiologiques.

2. Lame porte-objet selon la revendication 1, caractérisée en ce que plusieurs capteurs (6 à 12) sont disposés en étant répartis sur la périphérie de la zone d'observation (3), de préférence selon un cercle et au voisinage de la zone d'observation (3).

3. Lame porte-objet selon la revendication 1 ou 2, caractérisée en ce que l'on prévoit sur la lame porte-objet (1) des bornes électriques ou des surfaces de contact (5) pour la saisie de valeurs de mesure, de signaux de mesure et/ou pour l'alimentation électrique des capteurs (6 à 12).

4. Lame porte-objet selon l'une quelconque des revendications 1 à 3, caractérisée en ce que les capteurs (6 à 12) sont au moins partiellement encastrés dans la surface de la lame porte-objet (1) et, de préférence, se raccordent avec effleurement avec cette dernière.

5. Lame porte-objet selon l'une quelconque des revendications 1 à 4, caractérisée en ce qu'au moins un capteur (6 à 12) est réalisé sous la forme d'un circuit intégré comportant des transistors à effet de champ.

6. Lame porte-objet selon l'une quelconque des revendications 1 à 5, caractérisée en ce que l'on prévoit, sur la surface de la lame porte-objet (1), des bandes conductrices (13) qui sont, par exemple, déposées par impression, par vaporisation et/ou au moins partiellement noyées dans la surface.

7. Lame porte-objet selon l'une quelconque des revendications 1 à 6, caractérisée en ce que la lame porte-objet (1) est essentiellement constituée de silicium jusqu'à la zone d'observation (3), en ce que les capteurs (6 à 12) sont des semi-conducteurs au silicium et sont intégrés dans le silicium de la lame porte-objet (1) et que la lame porte-objet (1) est constituée par une matière optiquement transparente dans la zone d'observation (3).

8. Lame porte-objet selon l'une quelconque des revendications 1 à 7, caractérisée en ce que les capteurs (6 à 12) sont protégés de la lumière et qu'à cet effet, on prévoit, en particulier, une couche non transparente, de préférence noire.

9. Lame porte-objet selon l'une quelconque des revendications 1 à 8, caractérisée en ce que la lame porte-objet comporte un microprocesseur, en particulier un processeur de signaux, pour le traitement et/ou l'évaluation des signaux du capteur.

10. Lame porte-objet selon l'une quelconque des revendications 1 à 9, caractérisée en ce que la lame porte-objet (1) peut être maintenue à température constante.

11. Lame porte-objet selon l'une quelconque des revendications 1 à 10, caractérisée en ce que la lame porte-objet (1) comporte un chauffage de la couche mince régulé en température et en ce que celui-ci est prévu de préférence sur une face extérieure de la lame porte-objet (1) qui est opposée à la zone d'observation (2).

12. Lame porte-objet selon l'une quelconque des revendications 1 à 11, caractérisée en ce que, pour la fixation de la lame porte-objet (1), on prévoit une attache (14).

13. Lame porte-objet selon l'une quelconque des revendications 1 à 12, caractérisée en ce que l'attache peut être maintenue à température constante et est reliée, en position d'utilisation, à la lame porte-objet (1) avec une bonne conduction thermique, de préférence au moyen d'une surface support (21) conductrice de la chaleur en contact avec la lame porte-objet (1) par sa face inférieure.

14. Lame porte-objet selon l'une quelconque des revendications 1 à 13, caractérisée en ce que l'attache (14) comporte un conduit de liquide (15) ou une paroi creuse destiné à un liquide de conditionnement et comportant une entrée de liquide (16) et une sortie de liquide (17).

15. Lame porte-objet selon l'une quelconque des revendications 1 à 14, caractérisée en ce que l'attache (14) comprend un chauffage électrique à régulation thermostatique.

16. Lame porte-objet selon l'une quelconque des revendications 1 à 15, caractérisée en ce que, pour la saisie des signaux électriques, en particulier des capteurs (6 à 12) et/ou pour l'alimentation électrique, on prévoit une pièce de contact (33) qui peut être posée ou pressée sur les surfaces de contact (5) de la lame porte-objet (1) et qui comporte des contacts antagonistes s'adaptant aux surfaces de contact (5) de la lame porte-objet (1).

17. Lame porte-objet selon l'une quelconque des revendications 1 à 16, caractérisée en ce que la lame porte-objet (1) est intégrée dans un boîtier normalisé de circuit imprimé DIL ou PLCC ou est reliée à ce boîtier.

18. Lame porte-objet selon l'une quelconque des revendications 1 à 17, caractérisée en ce que l'on prévoit une fermeture de chambre (23) qui entoure sur le côté de manière étanche la zone de prise de vue (2) de la lame porte-objet (1) et délimite, en coopération avec cette dernière, une chambre de culture (27) sensiblement fermée et en ce que la fermeture de chambre (23) comporte une fenêtre (26) d'observation et/ou d'éclairage et présente au moins une ouverture d'entrée (28) ainsi qu'au moins une ouverture de sortie (29) pour un liquide nutritif.

19. Lame porte-objet selon l'une quelconque des revendications 1 à 18, caractérisée en ce que la fenêtre (26) est réalisée en une seule pièce avec la fermeture de chambre (23) et est constituée, de préférence, par 'une matière synthétique biologiquement inerte et fortement transparente.

20. Lame porte-objet selon l'une quelconque des revendications 1 à 19, caractérisée en ce que la fenêtre est disposée dans un évidement prévu dans la face supérieure de la fermeture de chambre (23).

21. Lame porte-objet selon l'une quelconque des revendications 1 à 20, caractérisée en ce que la fermeture de chambre (23) comporte des évidements de réception (31) dans lesquels des capteurs supplémentaires (32) comportant des supports de capteur peuvent être placés de manière amovible, de préférence vissés ou insérés.

22. Lame porte-objet selon l'une quelconque des revendications 1 à 21, caractérisée en ce que des capteurs à couche mince ou à structure plane sont prévus sur une paroi de la fermeture de chambre (23) qui est dirigée vers la chambre (27)

23. Lame porte-objet selon l'une quelconque des revendications 1 à 22, caractérisée en ce que, pour fermer les ouvertures de réception (31) non utilisées, on prévoit des bouchons de fermeture.

24. Lame porte-objet selon l'une quelconque des revendications 1 à 23, caractérisée en ce que la lame porte-objet (1) comporte un bord de maintien (22) qui s'étend à la périphérie de la zone de prise de vue (2) en faisant saillie vers le haut et qui entoure une ouverture qui s'étend jusqu'à la zone de prise de vue (2) et en ce que la fermeture de chambre (23) est placée de manière amovible dans l'ouverture.

25. Lame porte-objet selon l'une quelconque des revendications 1 à 24, caractérisée en ce que la lame porte-objet (1) est essentiellement réalisée sous la forme d'une vitre rectangulaire et en ce que la zone de prise de vue (2) est prévue sur une face plate (4) de la vitre.

26. Lame porte-objet selon l'une quelconque des revendications 1 à 25, caractérisée en ce que, sur la lame porte-objet (1), de préférence sur sa face supérieure, au voisinage des bords des petits côtés, on prévoit au moins un évidement de réception (34) pour l'insertion d'une pièce de contact.

27. Lame porte-objet selon l'une quelconque des revendications 1 à 26, caractérisée en ce que la zone de prise de vue (2) comporte, pour la culture de cellules non adhérentes, une structure plane supérieure, en particulier une structure en lamelles ou à grille.
